# EUROPÄISCHE PATENTSCHRIFT

(11) **EP 0 801 942 B1**
(45) Veröffentlichungstag und Bekanntmachung des Hinweises auf die Patenterteilung: **14.10.1998**
(21) Anmeldenummer: 97104820.2
(22) Anmeldetag: 21.03.1997
(51) Int. Cl.: A61K 7/13, A61K 7/06

(54) **Mittel zur Färbung und Tönung von menschlichen Haaren**
Composition for dyeing and tinting of human hair
Composition pour colorer et teindre les cheveux humains

(30) Priorität: 20.04.1996 DE 19615821
(43) Veröffentlichungstag der Anmeldung: 22.10.1997
(73) Patentinhaber: GOLDWELL GmbH, D-64297 Darmstadt (DE)
(72) Erfinder: Lorenz, Heribert, 64401 Gross-Bieberau (DE); Petzke, Erika, 64319 Pfungstadt (DE); Klusch, Hans, 64319 Pfungstadt (DE)

(56) Entgegenhaltungen:
- EP-A- 0 301 197
- WO-A-93/16678
- LU-A- 53 850
- PATENT ABSTRACTS OF JAPAN vol. 017, no. 674, 10.Dezember 1993 & JP 05 221840 A (KANEBO), 31.August 1993,
- DATABASE WPI Week 9423 Derwent Publications Ltd., London, GB; AN 94-188867 XP002037384 "Cosmetics containing aspalachus linearis extract to give wetness and moisture-remaining property to skin and hair" & JP 06 128 121 A (SANSEI SEIYAKU) , 10.Mai 1994

## Beschreibung

Die vorliegende Erfindung betrifft ein Mittel zum Färben bzw. Tönen von menschlichen Haaren mit verbesserten Gebrauchseigenschaften.

Es ist allgemein bekannt, daß Haarfärbemittel in zwei Kategorien aufgeteilt werden, nämlich einerseits die permanenten Haarfärbemittel, die grundsätzlich Haarfarbstoffvorprodukte enthalten, die zusammen mit Oxidationsmitteln je nach Zusammensetzung die gewünschte Färbung auf dem Haar entwickeln; und andererseits semipermanente Haarfärbemittel, die direktziehende Farbstoffe enthalten, die zur Entwicklung ihrer Färbeleistung keinerlei Oxidationsmittelzusatzes bedürfen. Dementsprechend sind die Färbungen auch weniger dauerhaft als diejenigen mit Permanentfarbstoffen erzielbaren.

Diese Farbzusammensetzungen auf Basis direktziehender Farbstoffe werden in der Regel entweder als Tönungsshampoos, als -lotionen oder als Tönungsfestiger, gegebenenfalls auch als Aerosolschaum, appliziert. Vereinzelt wurde, insbesondere für Färbemittel auf Basis von pflanzlichen Farbstoffen, auch bereits die Verwendung von pulverförmigen Produkten vorgeschlagen, die vor ihrer Anwendung auf dem Haar mit Wasser angerührt werden müssen.

Alle diese Zusammensetzungen weisen den Nachteil auf, daß sie bei der Anwendung nicht nur bestimmungsgemäß das Haar, sondern auch die Kopfhaut und die benachbarten Teile der Gesichtshaut färben.

WO 93/16678 betrifft ein Hautschutzmittel, enthaltend Polyethylenglykol mit einem mittleren Molekular gewicht von 2700 - 7500, hydrierts Risinusöl

und Glycerin und/oder Ethylhexandiol. Diese Zusammensetzung wird jedoch nicht im Haarfärbemittel selbst angewandt, sondern vor der Haarfärbung aufgebracht. Die vorliegende Erfindung geht daher von der Aufgabenstellung aus, ein Haarfarbemittel auf Basis direktziehender Farbstoffe zu schaffen, das diese Nachteile nicht aufweist.

Diese Aufgabe wird erfindungsgemäß dadurch gelöst, daß man einem Haarfärbe- bzw. -tönungsmittel das mindestens einen direktziehenden kationischen Haarfarbstoff enthält, ein Polyethylenglykol und/oder ein Polypropylenglykol, vorzugsweise mit einem Molekulargewicht zwischen etwa 150 und etwa 50 000, zusetzt.

Die Erfindung umfaßt auch die Verwendung von Polyethylenglykol bzw. Polypropylenglykol zur Verbinderung bzw. Verminderung der Hautanfärbung in Mitteln zum Färben und Tönen von menschlichen Haaren auf Basis mindestens eines direktziehenden Haarfarbstoffs.

Die bevorzugte Menge an Polyethylenglykol (PEG) bzw. Polypropylenglykol (PPG) in den erfindungsgemäßen Haarfärbemitteln liegt bei etwa 0,5 bis etwa 10, vorzugsweise 1 bis 9, insbesondere bei 2,5 bis 7,5 Gew.-%, berechnet auf die Gesamtzusammensetzung des anwendungsfertigen Haarfärbemittels (d. h., bei Konzentraten wie Pulvern bezieht sich die Angabe selbstverständlich auf das unmittelbar vor der Anwendung mit Wasser verdünnte Produkt. Die Konzentration im Pulver liegt dann natürlich entsprechend höher). Es können natürlich auch Gemische aus PEG und PPG eingesetzt werden.

Durch die Mitverwendung von PEG bzw. PPG wird nicht nur eine Verminderung bzw. Vermeidung der Anfärbung der Haut erreicht; überraschenderweise tritt auch keine negative Beeinflussung der Haarfärbewirkung ein.

Als direktziehende Haarfarbstoffe können im Prinzip alle für diesen Zweck zugelassenen Farbstoffe verwendet werden; es wird hierzu insbesondere auf die deutsche "Verordnung über kosmetische Mittel (Kosmetik-Verordnung)" in der jeweils geltenden Fassung, Anlage 3, verwiesen.

Bevorzugt sind jedoch die kationischen (basischen) Farbstoffe. Besonders geeignete basische (kationische) Farbstoffe sind:
- Basic Blue 6,: C.I.-No. 51,175;
- Basic Blue 7,: C.I.-No. 42,595;
- Basic Blue 9,: C.I.-No. 52,015;
- Basic Blue 26,: C.I.-No. 44,045;
- Basic Blue 41,: C.I.-No. 11,154;
- Basic Blue 99,: C.I.-No. 56,059;
- Basic Brown 4,: C.I.-No. 21,010;
- Basic Brown 16,: C.I.-No. 12,250;
- Basic Brown 17,: C.I.-No. 12,251;
- Basic Green 1,: C.I.-No. 42,040;
- Basic Red 2,: C.I.-No. 50,240;
- Basic Red 22,: C.I.-No. 11,055;
- Basic Red 76,: C.I.-No. 12,245;
- Basic Violet 1,: C.I.-No. 42,535;
- Basic Violet 3,: C.I.-No. 42,555;
- Basic Violet 10,: C.I.-No. 45,170;
- Basic Violet 14,: C.I.-No. 42,510;
- Basic Yellow 57,: C.I.-No. 12,719;

Als mögliche saure (anionische) Farbstoffe gemaß dem Verwendungsanspruch 4 können auch Verwendung finden:
- Acid Black 1,: C.I.-No. 20,470;
- Acid Blue 9,: C.I.-No. 42,090;
- Acid Blue 74,: C.I.-No. 73,015;
- Acid Red 18,: C.I.-No. 16,255;
- Acid Red 27,: C.I.-No. 16,185;
- Acid Red 87,: C,I.-No. 45,380;
- Acid Red 92,: C.I.-No. 45,410;
- Acid Violet 43,: C.I.-No. 60,730;
- Acid Yellow 1,: C.I.-No. 10,316;
- Acid Yellow 23,: C.I.-No. 19,140;
- Acid Yellow 3,: C.I.-No. 47,005;
- D&C Brown No.1,: C.I.-No. 20,170;
- D&C Green No.5,: C.I.-No. 61,570;
- D&C Orange No.4,: C.I.-No. 15,510;
- D&C Orange No.10,: C.I.-No. 45,425:1;
- D&C Orange No.11,: C.I.-No. 45,425;
- D&C Red No.21,: C.I.-No. 45,380:2;
- D&C Red No.27,: C.I.-No. 45,410:1;
- D&C Red No.33,: C.I.-No. 17,200;
- D&C Yellow No.7,: C.I.-No. 45,350:1;
- D&C Yellow No.8,: C.I.-No. 45,350;
- FD&C Red No.4,: C.I.-No. 14,700;
- FD&C Yellow No.6,: C.I.-No. 15,985.

Auch pflanzliche Farbstoffe können allein oder in Kombination mit synthetischen Direktziehern Verwendung finden, beispielsweise Henna (rot oder schwarz), Alkannawurzel, Laccainsäure (Stocklack), Blauholzpulver, Krappwurzel- und Rhabarberwurzelpulver, etc.

Der Anteil der direktziehenden Farbstoffe in den erfindungsgemäßen Zusammensetzungen ist je nach gewünschtem Farbton und Applikationsform variabel; sie liegt bei pulverförmigen Produkten, die bei ihrer Anwendung erheblich mit Wasser verdünnt werden, im allgemeinen zwischen etwa 0,1 bis etwa 25, vorzugsweise 0,5 bis 10, insbesondere 1 bis 5 Gew.-% des Mittels.

Liegt das Mittel als Lösung, Dispersion, Emulsion, Gel oder Aerosolpräparat zur direkten Anwendung, d. h., ohne vorherige Verdünnung, vor, so liegt die Einsatzkonzentration entsprechend niedriger, vorzugsweise bei etwa 0,005 bis etwa 2,5 Gew.-% des Färbemittels, vorzugsweise 0,01 bis 1,5 Gew.-%, insbesondere etwa 0,05 bis 1 Gew.-%, besonders bevorzugt 0,1 bis 0,5 Gew.-% der Gesamtzusammensetzung. Die als Grundlage für die erfindungsgemäßen Haarfärbemittel in Betracht kommenden Zusammensetzungen sind an sich bekannt; es wird, beispielsweise, auf die bekannte Monographie von K. Schrader, Grundlagen und Rezepturen der Kosmetika, 2. Aufl. (1989), S. 800 bis 815, verwiesen.

Bei den erfindungsgemäß verwendeten Polyethylenglykolen und Polypropylenglykolen handelt es sich um handelsübliche Produkte innerhalb eines weitgespannten Molekulargewichtsbereichs zwischen etwa 150 und etwa 50 000, vorzugsweise zwischen 250 und 35 000, besonders bevorzugt zwischen 1000 und 25 000, insbesondere 2500 bis 20 000.
Gleiches gilt für Polypropylenglykol.

Geeignete Handelsprodukte sind beispielsweise die verschiedenen "Polywachs®"-Typen, z. B. "Polywachs® 550", "Polywachs® 650", "Polywachs® 1000", "Polywachs® 1550", "Polywachs® 4000", "Polywachs® 5000/6000", und "Polywachs® 12000" oder "Carbowaxe®".

Eine Übersicht findet sich z. B. bei Schrader, l.c., S. 234 - 235 sowie bei H. P. Fiedler, Lexikon der Hilfsstoffe für Pharmazie, Kosmetik und angrenzende Gebiete, 3. Aufl. (1989), S. 964 - 969 .

Entsprechende Polypropylenglykole sind ebenfalls bekannt, vgl. Fiedler, l.c., S. 990 - 991, z. B. die Handelsprodukte "Polyglycol P®" der Dow Chemical Co. oder "Witconol®" der Witco Co.

Das Haarfärbe- und Tönungsmittel nach der Erfindung kann mindestens ein synthetisches oder natürliches haarkonditionierendes Polymeres, vorzugsweise in einer Menge von 0,1 bis 10, insbesondere 0,5 bis 5 Gew.-% der Gesamtzusammensetzung, enthalten. Obwohl grundsätzlich alle Arten von Polymeren verwendet werden können, also nichtionische, anionische, amphotere und kationische Polymere, werden kationische Polymere im Rahmen der Erfindung bevorzugt.

Handelt es sich bei den erfindungsgemäßen Produkten um Farbpulver, so enthalten diese üblicherweise eine in Wasser lösliche oder zumindest quellbare Pudergrundlage, beispielsweise Stärke, Maltodextrin, Cyclodextrin, Silicagel, Polyethylenglykole, etc., vorzugsweise in einer Menge von etwa 10 bis etwa 70, insbesondere etwa 20 bis etwa 50 Gew.-% der Gesamtzusammensetzung.

Diese Bestandteile sind ebenso bekannt wie die Mitverwendung von Tensiden, insbesondere, wenn es sich um Tönungsshampoos handelt, vorzugsweise in einer Menge von etwa 5 bis 30 Gew.-% des Mittels.

Geeignete Tenside sind anionische Tenside wie langkettige N-Acylaminocarbonsäuren und deren Salze wie N-Lauroylsarkosinat und -glutamat, amphotere Tenside wie Betaine, z. B. Cocoamidopropylbetain, sowie nichtionische und kationische Tenside, z. B. C₁₂-C₁₈-Alkylpolyglykolether, und Ethylenoxid/ Propylenoxid-Copolykondensate und langkettige quaternäre Ammoniumverbindungen, Z. B. Distearyl- oder Dilauryldimethylammoniumchlorid.

In Farbemulsionen bzw. -dispersionen finden vorzugsweise kationische Tenside Verwendung.

Die erfindungsgemäßen Färbemittel können die in solchen Zusammensetzungen üblichen Zusätze enthalten, deren Art und Charakter von der Applikationsform des Mittels abhängig ist, z. B. Fette, Fettalkohole, Emulgatoren, pH-Regulatoren, Eiweißhydrolysate, Lösungsvermittler, Konservierungsmittel, Parfums, etc.

Handelt es sich um Farbpulver, werden diese vor ihrer Anwendung mit Wasser im Verhältnis von etwa 1 : 5 bis etwa 1 : 30, vorzugsweise 1 : 10 bis 1 : 20, angerührt, auf dem Haar gleichmäßig verteilt und nach etwa 20- bis 30-minütiger Einwirkung durch Shampoonieren entfernt.

Bei Lösungen, Gelen, Emulsionen, Dispersionen, etc. erfolgt die Aufbringung direkt auf das zu färbende Haar.

Die folgenden Beispiele illustrieren die Erfindung und die damit erzielten Effekte.

### Beispiel 1

| Gel | |
|---|---|
| Guar Gum | 1,00 (Gew.-%) |
| Stärke | 0,25 |
| Honigextrakt | 0,15 |
| Weizenprotein-Hydrolysat | 0,30 |
| Kaliumsorbat | 0,15 |
| Basic Blue 99 | 0,03 |
| Basic Brown 16 | 0,18 |
| Basic Brown 17 | 0,01 |
| Basic Red 2 | 0,01 |
| PEG-20 (Polyethylenglykol; MG 950 - 1050) | 5,90 |
| Wasser | @ 100,00 |

### Beispiel 1A

Analog Beispiel 1, jedoch ohne Polyethylenglykol 1000.

Beide Produkte ergaben eine helle rotbraune, nicht zu unterscheidende Haarfärbung im Halbseitenversuch.

Die hautanfärbende Wirkung beider Produkte wurde dadurch verglichen, daß sie bei 8 Versuchspersonen auf die Fingerkuppen jeweils einer Hand aufgetragen wurden. Nach 2 Minuten wurden die Hände mit warmem Wasser gewaschen.

Dabei ergab sich bei allen 8 Versuchspersonen, daß die Fingerkuppen der mit einem Gel nach Beispiel 1 behandelten Hände weitgehend entfärbt waren, während auf den Fingerkuppen der mit einem Gel nach Beispiel 1A behandelten Hände eine deutliche Rot-Braunfärbung zurückblieb.

### Beispiel 2

| Farbemulsion | |
|---|---|
| Oleth-5 | 0,85 (Gew.-%) |
| Cetearylalkohol | 0,85 |
| Stearamide MEA | 0,15 |
| Cocoamide MEA | 0,15 |
| Hydroxyethylcellulose | 0,30 |
| EDTA | 0,10 |
| Ammoniumchlorid | 0,25 |
| Ethanolamin | 0,02 |
| Panthenol | 0,20 |
| Cocoamidopropylbetain | 2,50 |
| Weizenproteinhydrolysat | 0,10 |
| Diethylenglykol | 0,30 |
| Roßkastanienextrakt | 0,15 |
| Hopfenextrakt | 0,05 |
| Parfum | 0,15 |
| Konservierungsmittel | 0,15 |
| PEG-200 (MG ∼9000) | 5,00 |
| Basic Blue 99 | 0,12 |
| Basic Brown 16 | 0,17 |
| Basic Brown 17 | 0,07 |
| HC Yellow 5 | 0,12 |
| Wasser | @ 100,00 |

Eine analoge Zusammensetzung 2A enthielt kein PEG-200.

Die Ausfärbung erfolgte an jeweils 8 Probandinnen im Halbseitenversuch, wie in Beispiel 1 beschrieben.

In beiden Fällen wurde eine dunkle rotbraune Färbung erhalten; bei den im Anfärbeversuch der Fingerkuppen mit der Zusammensetzung nach Beispiel 2A behandelten Probanden ergab sich durchweg eine Anfärbung, im Gegensatz zu denjenigen mit der Zusammensetzung nach Beispiel 2.

### Beispiel 3

| Pulver | |
|---|---|
| Cyclooctaamylose | 4,8 (Gew.-%) |
| Guar Hydroxypropyltrimoniumchlorid | 12,5 |
| PPG-9 (Polypropylenglykol; MG ∼400) | 60,5 |
| Kaliumsorbat | 1,8 |
| Weizenproteinhydrolysat | 5,0 |
| Nichtionischer Emulgator | 12,0 |
| Basic Brown 16 | 0,8 |
| Basic Brown 17 | 0,8 |
| Basic Yellow 57 | 0,3 |
| Basic Blue 99 | 1,5 |

Ein Beispiel 3A enthielt kein PPG; anstelle dessen Diatomeenerde.

Die Pulver wurden vor der Anwendung jeweils im Verhältnis 5 g Pulver mit 80 ml Wasser angerührt und die erhaltenen verdickten Lösungen, wie in Beispiel 1 beschrieben, im Halbseitenversuch auf das Haar von 8 Probandinnen aufgebracht und nach 15-minütiger Einwirkung ausgespült.
Es wurde eine nicht unterscheidbare naturbraune Haarfärbung erhalten.

Der analog Beispiel 1 durchgeführte Fingerkuppen-Anfärbungsversuch zeigte eine durchgehende Färbung mit der Zusammensetzung mit Beispiel 3A und keine Anfärbung mit derjenigen nach Beispiel 3.

## Patentansprüche

1. Mittel zur Färbung und Tönung von menschlichen Haaren auf Basis mindestens eines direktziehenden kationischen Haarfarbstoffs, enthaltend mindestens ein Polyethylenglykol und/oder Polypropylenglykol.

2. Mittel nach Anspruch 1, dadurch gekennzeichnet, daß das Molekulargewicht des Polyethylenglykols und/oder Polypropylenglykols zwischen 150 und 50 000 liegt.

3. Mittel nach Anspruch 2, dadurch gekennzeichnet, daß das Molekulargewicht des Polyethylenglykols und/oder Polypropylenglykols zwischen 250 und 35 000 liegt.

4. Verwendung von Polyethylenglykol und/oder Polypropylenglykol zur Verhinderung bzw. Verminderung der Hautanfärbung in Mitteln zum Färben und Tönen von menschlichen Haaren auf Basis mindestens eines direktziehenden Haarfarbstoffs.

## Claims

1. Composition for dyeing of human hair on the basis of at least one direct-acting hair dyestuff, comprising at least one polyethylene glycol and (or) polypropylene glycol.

2. Composition according to claim 1, containing a polyethylene glycol and (or) polypropylene glycol molecular weight between 150 and 50,000.

3. Composition according to claim 2, containing a polyethylene glycol and (or) polypropylene glycol molecular weight between 250 and 35,000.

4. Use of polyethylene glycol and (or) polypropylene glycol to prevent or reduce skin staining in compositions based on direct-acting dyestuffs dyeing of human hair.

## Revendications

1. Composition pour colorer et teindre des cheveux humains à base d'au moins un colorant pour cheveux direct cationique, contenant au moins un polyéthylène glycol et/ou un polypropylène glycol.

2. Composition selon la revendication 1, caractérisée en ce que la masse moléculaire du polyéthylène glycol et/ou du polypropylène glycol est comprise entre 150 et 50 000.

3. Composition selon la revendication 2, caractérisée en ce que la masse moléculaire du polyéthylène glycol et/ou du polypropylène glycol est comprise entre 250 et 35 000.

4. Utilisation de polyéthylène glycol et/ou de polypropylène glycol pour empêcher et/ou pour atténuer la coloration de la peau dans des compositions pour colorer et teindre des cheveux humains à base d'au moins un colorant pour cheveux direct.
